# EUROPEAN PATENT APPLICATION

(11) **EP 4 194 440 A1**
(43) Date of publication of application: **14.06.2023**
(21) Application number: 21213611.3
(22) Date of filing: 10.12.2021
(51) Int. Cl.: C07D 207/12, C07K 4/00, B01J 31/00

(54) **ATOM TRANSFER RADICAL REACTION USING A PEPTIDE-BASED METAL CATALYST AND SELF-ASSEMBLY OF PEPTIDES AND METAL ION**

(71) Applicant: Adolphe Merkle Institute, University of Fribourg, 1700 Fribourg (CH); Université de Strasbourg, 67000 Strasbourg (FR); CENTRE NATIONAL DE LA RECHERCHE SCIENTIFIQUE (CNRS), 75016 Paris (FR)
(72) Inventor: Pellizzoni, Michela Maria, 1700 Fribourg (CH); Lebrun, Vincent, 67100 Strasbourg (FR)
(74) Representative: P&TS SA (AG, Ltd.)

(57) **Abstract**

The present invention relates to a process of reacting an halogenated compound and an unsaturated compound through an Atom Radical Transfer reaction, in the presence of a self-assembly (1) of at least two peptides and a metal ion (M) wherein at least one of the peptides comprises a metal coordination moiety (21, 31) adapted to bound a metal ion (M) and wherein the peptides comprise charges amino acids (22, 32) arranged so as to form and maintain said self-assembly (1) at a pH comprised between about 6 and about 8 in an aqueous media. The present invention further relates to a self-assembly used as a peptide-based catalyst for such an Atom Radical Transfer reaction in an aqueous media.

## Description

### Technical domain

The present invention concerns a process for reacting an halogenated compound and an unsaturated compound through an Atom Transfer Radical reaction, in the presence of a self-assembly of peptides and metal ion, in particular cupper ion. The reaction can be performed in water, and in particular in non-degassed water at mild temperatures. Very good or complete chemoselectivity is obtained. Enantioselectivity is also obtained. It further concerns the corresponding self-assembly..

### Related art

Document WO2004096872 discloses complexes of transition metals radical reactions such as atom transfer radical polymerisation (ATRP), atom transfer radical addition (ATRA), atom transfer radical cyclisation (ATRC), and aims at predicting and evaluate some transition metal complexes for particular selective catalytic reactions. The transition metal complexes here disclosed are based on traditional small bidentate ligands.

The article *"*Peptide-Based catalyst reach the outer sphere through remote desymmetrisation and atroposelectivity", Acc.Chem.Res. 2019, 52, 199-215, examines the use of peptides as chiral ligands in chemical reactions.

The article *"*Amyloid inspired Self-Assembly peptides nanofibers", Biomacromolecules 2012, 13, 3377-3387, discloses the self-arrangement of peptides used for encapsulation of zwitterionic dyes and drugs for potential medical applications.

It appears that radical based reactions, and in particular ATRC, ATRA and ATRP, mostly remain performed under organic solvent conditions and/or at high temperatures. These conditions still do not allow a large variety of radical reactions under biological conditions, meaning in aqueous media at temperatures lower than 40°C and at pH physiologically acceptable. Usually, the conventional catalysts lose their activity under such mild and biocompatible conditions. In fact ATRC could be conducted in water but some side reactions can occur and contribute to an inefficient radical generation. Water can cause the disproportionation of the metal-ligand complex. For example, disproportionation of Cu (I) halide complex produce Cu(0), Cu(ll) complex and the free ligand. Another side reaction that occurs to a significant extent in water is the hydrolysis of the metal-halide-ligand complex. Furthermore, water can solvate the halide of Cu(II)-halide complex and then coordinate to the metal, preventing the proper reaction to occur. There is thus room for improving the reaction conditions of radical transfer based reactions such as ATRP, ATRC, and ATRA.

### Short disclosure of the invention

An aim of the present invention is the provision of a process and conditions adapted to perform atom transfer radical reactions such as ATRP, ATRA, ATRC under mild and biocompatible conditions. It is in particular an object to perform catalysed atom transfer radical reactions in aqueous media at temperatures below 40°C, preferably below 30°C. It is in particular an aim of the present invention to provide a process adapted to perform atom transfer radical reactions such as ATRP, ATRA, ATRC in an aqueous solvent free or substantially free of organic solvent.

Another aim of the invention is the provision of a self-assembly of peptides together with a metal ion.

Another aim of the present invention is the use of such a self-assembly of peptides and metal ion as a stable and efficient catalyst for atom transfer radical reactions, in particular for ATRP, ATRA, ATRC under mild and biocompatible conditions.

Another aim of the present invention is to perform an atom transfer radical reactions such as ATRP, ATRA, ATRC with an improved chemoselectivity and/or stereoselectivity.

Another aim of the present invention is to provide a process and a catalyst adapted to produce enantioenriched lactams, such as β-lactams, γ-lactams and other lactams in an aqueous media.

According to the invention, these aims are attained by the object of the independent claims, and further detailed through the claim depending thereof.

With respect to what is known in the art, the invention provides the advantage to perform a large variety of atom transfer radical reactions under biological-like conditions.

### Short description of the drawings

Exemplar embodiments of the invention are disclosed in the description and illustrated by the following drawings :
- Figure 1 : Schematic representation of a self-assembly according to an example of the present invention
- Figure 2a : Schematic representation of a self-assembly according to another example of the present invention
- Figure 2b: Schematic representation of a self-assembly according to another example of the present invention

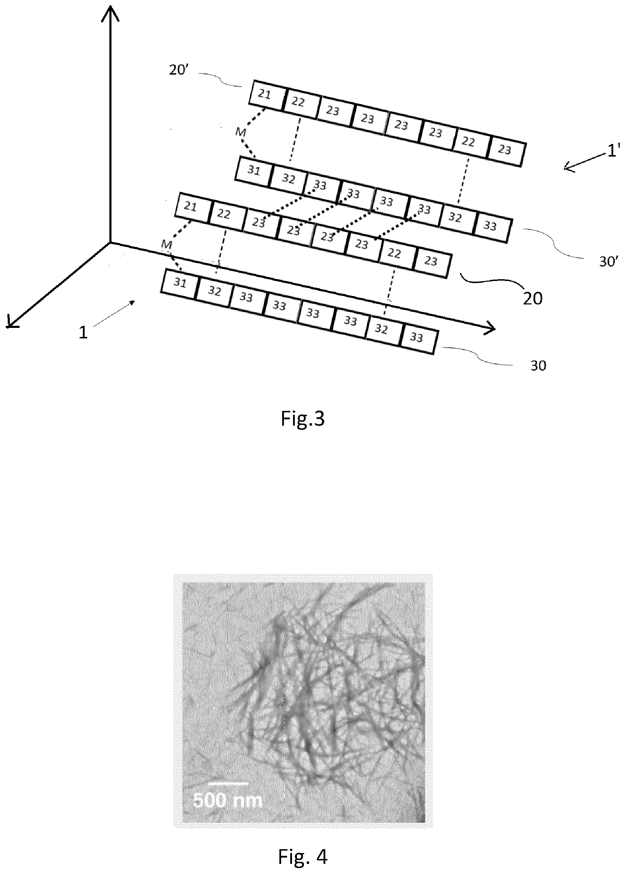

- Figure 3 : Schematic representation of a self-assembly according to the present invention
- Figure 4: Macroscopic view of the self-assembly according to the present invention.

### Examples of embodiments of the present invention

The present description relates to an Atom Transfer Radical reaction between an halogenated compound and a unsaturated compound. Such Atom Transfer Radical reaction can be an Atom Transfer Radical Cyclisation (ATRC). In this particular case, each one of the halogenated compound and the unsaturated compound denotes a part of one same molecule, and react together to cyclise the molecule. The Atom Transfer Radical reaction according to the present description can alternatively be an Atom Transfer Radical Polymerization (ATRP) or an Atom Transfer Radical Addition (ATRA) between two distinct compounds.

The halogenated compound comprises an halogen atom such as fluorine (F), Bromine (Br), chlorine (Cl), and iodine (I), preferably one of Cl, Br and I, more preferably Cl or Br. The halogen atom is preferably at a position Alpha to a double bond comprising an heteroatom selected from oxygen, nitrogen, sulfur. Such a double bond is preferably a CO double bond. The halogen atom is thus in position alpha to a ketone, a sulfone, an amide, a carboxy, a sulfoxy, an imine, or other related chemical group. Is is preferably in position alpha to a ketone or an amide.

Such halogenated compound can be a compound of Formula P10 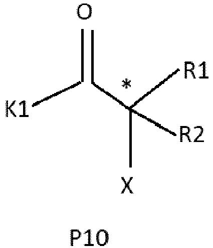 Wherein
- X denotes an halogen atom such as fluorine, bromine, chlorine or iodine, preferably fluorine, bromine or chlorine, most preferably, bromine or chlorine.
- R₁ and R₂ independently from each other denote hydrogen, an halogen atom selected from fluorine, chlorine, bromine, iodine, preferably, from chlorine or bromine, a linear or branched saturated or unsaturated alkyl group having from 1 to 10 carbon atoms, preferably from 1 to 5 carbon atoms, optionally substituted by 1 to 3 groups independently selected from OR₃, NR₃R_{3'}, SR₃, an aryl group, an heteroaryl group or a saturated or unsaturated cycloalkyl group having from 3 to 7 carbon atoms.
- R₃, R_{3'} independently from each other denote H, a linear or branched saturated or unsaturated alkyl group having from 1 to 10 carbon atoms, preferably from 1 to 5 carbon atoms, optionally substituted by 1 to 3 groups independently selected from an aryl group, an heteroaryl group or a saturated or unsaturated cycloalkyl group having from 3 to 7 carbon atoms.
- K1 denotes one of OR₃, NR₃R_{3'}, or SR₃, a linear or branched saturated or unsaturated alkyl group having from 1 to 10 carbon atoms, preferably from 1 to 5 carbon atoms, optionally substituted by 1 to 3 groups independently selected from OR₃, NR₃R_{3'}, and SR₃ and wherein one of the carbon atom of said linear or branched saturated or unsaturated alkyl group is optionally replaced by an oxygen or a sulphur atom or by a group NR₃
- The star (*) denotes a chiral centre where the corresponding substituents are all different.

The unsaturated compound denotes a compound comprising at least one double bond between two adjacent carbon atoms. Such a carbon-carbon double bond can be terminal, meaning that one of its carbon atom only bears hydrogen atoms. Alternatively, the terminal carbon atom of the carbon-carbon double bond bears one hydrogen atom and a substituent selected from an aryl group, preferably a phenyl, or an alkyl group comprising from one to 5 carbon atoms, preferably one of a methyl, ethyl or propyl group. The carbon-carbon double bond, when both of its carbon atoms are substituted, can have a cis or a trans configuration. The unsaturated compound can be a compound of formula **P10'** 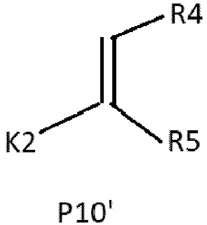 Wherein
- K2 denotes one of OR₃, NR₃R_{3'}, SR₃, a linear or branched saturated or unsaturated alkyl group having from 1 to 10 carbon atoms, preferably from 1 to 5 carbon atoms, optionally substituted by 1 to 3 groups independently selected from OR₃, NR₃R_{3'}, and SR₃ and wherein one of the carbon atom of said linear or branched saturated or unsaturated alkyl group is optionally replaced by an oxygen or a sulphur atom or by a group NR₃
- Each of R₄ and R₅ independently from each other denotes an aryl group or an alkyl group having from 1 to 5 carbon atoms, preferably one of a methyl, ethyl or propyl group.

The resulting compound is one of the compounds of formula **P20** and **P20'** or a mixture thereof 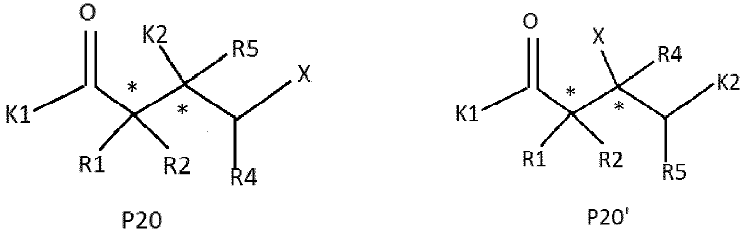 Wherein K₁, K₂, R₁, R₂, R₃, R₄, R₅, X and the star (*) are as defined above.

According to an embodiment, **K1** and **K2** form together a link **L** between the halogenated compound and the unsaturated compound so as to form a single compound of formula **P10"**

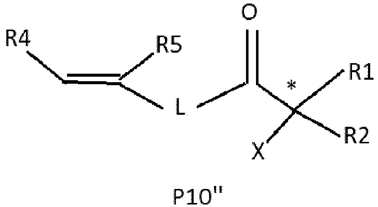

Wherein
- **L** denotes a linear or branched saturated or unsaturated alkyl group having from 1 to 10 carbon atoms, preferably from 1 to 5 carbon atoms, optionally substituted by 1 to 3 groups independently selected from OR₃, NR₃R_{3'}, and SR₃, and/or wherein one of the carbon atom of said linear or branched saturated or unsaturated alkyl group is replaced by an oxygen or a sulphur atom or by a group NR₃,
- wherein X, R₁, R₂, R₃, R_{3'}, R₄ and R₅ are has above defined.

Examples of compounds of formula **P10"** are as follows:

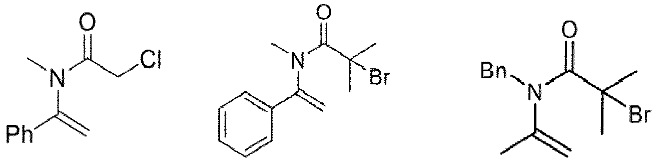

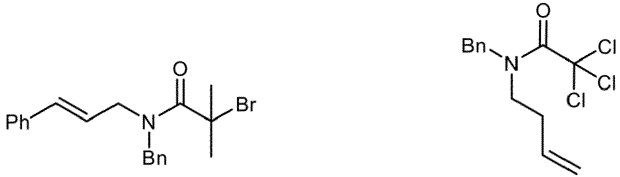

According to such an embodiment the Atom Transfer Radical reaction preferably denotes an Atom Transfer Radical Cyclisation (ATRC). The resulting compound is one of a compound of formula **P21** and **P22** or a mixture thereof. 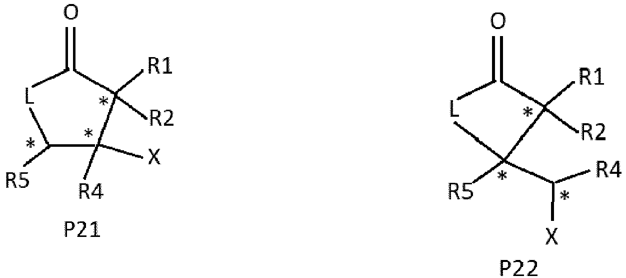 Wherein L, X, R₁, R₂, R₃, R₄ and R₅ are has above defined.

For the purpose of the present description an *"aryl"* or an *"aryl group"* denotes a monocyclic, bicyclic or tricyclic carbon aromatic group. Examples of aryl groups are phenyl, naphthalene, anthracene, phenanthrene, azulene.

For the purpose of the present description an *"heteroaryl"* or an *"heteroaryl group"* denotes a monocyclic, bicyclic or tricyclic aromatic group comprising from 1 to 3 heteroatom independently selected from Nitrogen, Oxygen and Sulphur. Example of heteroaryl groups are furan, thiophen, pyrrole, pyridine, indole, imidazole, purine, quinoline, quinoxaline.

For the purpose of the present description, a saturated alkyl denotes an alkyl exempt from double and triple bonds. An unsaturated alkyl denotes an alkyl comprising one or more double bond or one or more triple bond. An unsaturated alkyl may comprise from 1 to 3 double bond, or from 1 to 3 triple bonds, or a mixture of 1 to 3 double and triple bonds.

The present Atom Transfer Radical reaction is performed in an aqueous solvent, comprising more than 70% or 80% of water, preferably more than 90% of water or more than 95 % of water. In a preferred arrangement, the solvent is only water. The water used as a solvent in the present Atom Transfer Radical reaction does not need to be degassed, meaning that the solvent can comprise oxygen solubilised from the atmosphere.

The pH is comprised between 6 and 8, preferably comprised between 6.5 and 7.5, such as a pH of 6.7 to 7. A buffer can be used to maintain the pH under requested values. For example tris(hydroxymethyl)aminomethane, also known as Tris, can be used as buffering additive. According to a preferred embodiment, the buffering agent is not degassed. The temperature is comprised between 25°C and 45°C, preferably around 30°C to 40°C.

The Atom Transfer Radical reaction of the present description is performed in the presence of a reducing agent and in the presence of a metal ion **M**, further described below. The reducing agent is added in the reaction in excess with regards to the metal ion **M**. An excess of reducing agent of 1 to 200 can be used compared to the metal ion **M**. It has been found that an excess of reducing agent from 1 to 30 compared to the metal ion M is in favour of the Atom Transfer Radical mechanism versus the reductive cyclisation or addition or polymerisation. An amount of reducing agent comprised between 1 and 5 compared to the metal ion **M** promotes the chemoselectivity of the 5-exo-trig ATRC. The metal ion is here preferably Cu (II).

Any suitable reductive agent may be used. For example sodium ascorbate can be used in the present reaction. The excess is given as molar ratios.

The Atom Transfer Radical reaction of the present description is performed in the presence of a self-assembly **1** of two or more peptides and a transition metal ion **M**. The self assembly 1 is stable in an aqueous media, such as pure water or a media comprising more than 70% or more than 80% water. A self-assembly **1** designates any spontaneous and stable arrangement of the peptides together with the metal ion **M**. Such self-assembly can then take the form of nanofibers, microfibers, sheets, such as β-sheets, α-helix, random coil and combination thereof. A self-assembly thus designates any tridimensional arrangement comprising at least one metal ion and two peptides which are linked together by chemical interactions such as polar or electrostatic bonds, hydrophobic, π-π interactions or a combination thereof to form a self-assembly **1**. This does not exclude that several self-assemblies **1** are arranged together to form a supramolecular arrangement. Depending on the conditions, a self-assembly **1** according to the present invention may form at the macroscopic scale, a suspension and/or gel, which is more or less viscous.

The metal ion **M** is selected among the transition metal ions. In particular it is selected among the transition metals commonly used for the catalysis of organic reactions, and more particularly for catalysing reaction based on radical transfer. Such metals are for example selected among Zinc, Palladium, Platinum, Copper, Silver and Nickel. Most preferably the metal ion **M** of the self-assembly **1** is copper.

The metal ion **M** included in the self-assembly **1** may be at any suitable oxidation level, such as (I), (II) or (III) depending on the selected metals and the conditions. According to a preferred embodiment, the metal **M** used in the present self-assembly **1** is copper at the oxidation levels (I) or (II), namely Cu (I) or Cu (II). Most preferably, the metal ion **M** is Cu (II).

The self-assembly **1**, comprises at least a first peptide **20** and a second peptide **30**. The metal ion **M** is bonded to one of the first **20** and second **30** peptides or to both of them. The term "bonded" is here understood as "combined" or "associated" in a way to form a stable self-assembly **1**. The corresponding bond may be of dative type, electrostatic type, of polar type or any other applicable type. A peptide may include from 2 to 20 amino-acids or from 3 to 15 amino-acids or from 4 to 10 amino-acids. The peptides of a given self-assembly **1** may have a different number of amino-acids or have the same number of amino-acids. The amino-acids of the peptides are preferably selected among the 20 natural L- amino-acids including Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophane (W), Tyrosine (Y) and Valine (V). One or more of the amino-acids may however be selected among the corresponding D-amino-acids. Alternatively or in addition, the lateral residue of one or more of the amino-acids may be modified or comprise substituents other than the natural residues of the amino-acids, such as an acetyl derivative. Both the first peptide **20** and the second peptide **30** of the self-assembly **1** comprises a N-terminal and a C-terminal extremity.

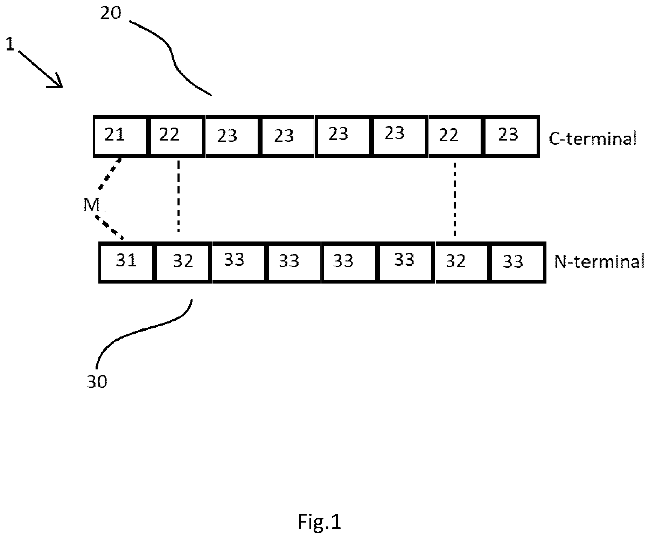

In a preferred arrangement, a self-assembly **1** comprises a first peptide **20**, a second peptide **30** and a metal ion **M**, wherein the metal ion **M** is bonded to one extremity of at least one of the first **20** and the second **30** peptide, preferably to one extremity of both first **20** and second **30** peptides either directly or through a metal coordination moiety (figure 1).

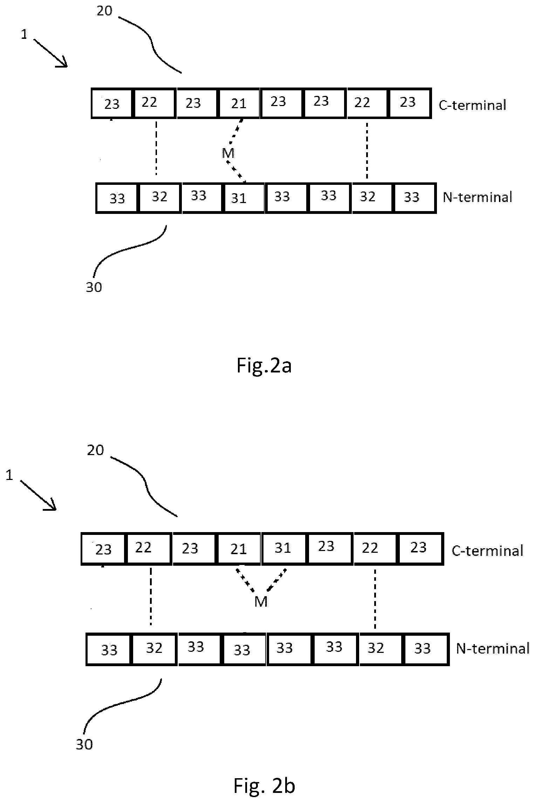

According to another embodiment, a self-assembly 1 comprises a first peptide 20, a second peptide 30 and a metal ion M, wherein the metal ion M is bonded to a central portion of at least one of the first 20 and the second 30 peptide, preferably to a central portion of both first 20 and second 30 peptides either directly or through a metal coordination moiety (figure 2a, 2b.).

Preferably, a self-assembly 1 according to the present description comprises a first peptide **20**, a second peptide **30** and a metal ion **M**, wherein the metal ion **M** is bonded to a central portion of only one of the first **20** and the second **30** peptide. In this particular case, an alpha helix configuration of the self-assembly **1** is privileged.

A metal coordination moiety denotes any chemical group or residue added to one extremity of a peptide or included in a central portion of the assembly **1**, which is adapted to bond to the metal ion **M**. In particular a coordination moiety comprises at least one donating heteroatom atom such as Nitrogen, Sulphur, Phosphorus, or Oxygen adapted to bond to a metal ion **M**, preferably from 1 to 3 or 1 or 2 such bonding heteroatoms. A metal coordinating moiety may comprise or not comprise additional heteroatoms which do not bind to the metal ion **M**. Alternatively, one or more donating heteroatom already present in the corresponding peptide may also be bond to the metal ion **M**. According to an embodiment, one donating heteroatom present on the amino-acid contiguous to the metal coordination moiety is bond to the metal ion **M**. The metal coordination moiety is preferably a small chemical entity comprising from 10 to 60 atoms, or from 10 to 40 atoms, or from 10 to 20 atoms. The metal coordination moiety is preferably linked to one of the extremities of the corresponding peptide through a peptide bond. A metal coordination moiety may comprise one or several chiral centres. Alternatively, it does not comprise any chirality. The donating heteroatoms of a metal coordination moiety are preferably selected from 1 or 2 nitrogen atoms, adapted for binding a copper ion such as Cu (I) or Cu (II) ion. Such nitrogen atoms may be included in a ring or not. A metal coordination moiety can be selected among natural amino-acids and/or non-natural amino-acids. Alternatively or in addition, a metal coordination moiety can denote any small chemical entity, which is not an amino-acid, and which is linked to an extremity of the corresponding peptide and which comprises such 1 or 2 nitrogen atoms available for bonding a Cu (I) or a Cu (II) ion.

The first peptide **20** thus comprises a first metal coordination moiety **21** at one of its N-terminal and C-terminal extremities. Said first metal coordination moiety **21** can consist of a terminal amino-acid of the first peptide **20**, provided that it comprises at least one nitrogen atom available for binding a metal ion **M**, such as a Cu (I) or a Cu (II) ion. Alternatively, the first metal coordination moiety **21** denotes a non-peptide chemical entity as defined above and linked to one of the C-terminal and N-terminal extremities of the first peptide **20**.

The second peptide **30** comprises a second metal coordinating moiety **31** at one of its N-terminal and C-terminal extremities. Said second metal coordination moiety **31** can consist of the terminal amino-acid of the second peptide **30**, provided that it comprises at least one nitrogen atom available for binding a metal ion **M**, such as a Cu (I) or a Cu (II) ion. Alternatively, the second metal coordination moiety **31** denotes a non-peptide chemical entity as defined above and linked to one of the C-terminal and N-terminal extremities of the second peptide **30**.

According to one embodiment, only one first metal coordination moiety **21** is present at one of the N-terminal and C-terminal extremities of the first peptide. Alternatively, both N-terminal and C-terminal extremities of the first peptide **20** can be provided with a metal coordination moiety **21**, either identical or different.

According to an embodiment, only one second metal coordination moiety **31** is present at one of the N-terminal and C-terminal extremities of the second peptide **30**. Alternatively, both N-terminal and C-terminal extremities of the first peptide **20** can be provided with a metal coordination moiety **31**, either identical or different. It is understood that both first peptide **20** and second peptide **30** can independently from each other bear 1 or 2 metal coordination moiety **21**, **31**.

The below description is directed to self-assemblies wherein the first **20** and the second **30** peptides are provided with only one metal coordination moiety. It can however be easily extrapolated to peptides having two metal coordination moieties at both extremities, where applicable.

According to a preferred embodiment, the first metal coordination moiety **21** is linked to one of the N-terminal and C-terminal extremities of the first peptide **20**, the other extremity remaining free of any metal coordination moiety, and the second metal coordinating moiety **31** is linked to the other one of the N-terminal and C-terminal extremities of the second peptide **30**, the other extremity remaining free of any metal coordination moiety. In other words, the first metal coordination moiety **21** is linked to the N-terminal extremity of the first peptide **20** while the second metal coordination moiety **31** is linked to the C-terminal extremity of the second peptide **30**, or the first metal coordination moiety **21** is linked to the C-terminal extremity of the first peptide **20** while the second metal coordination moiety **31** is linked to the N-terminal extremity of the second peptide **30**. In this particular case, the first **20** and the second **30** peptides can arrange themselves in antisense with regard to the metal ion **M** to which there are bonded.

This does not fully exclude that both first **20** and second **30** peptides are both provided with one metal coordination moiety **21**, **31** at their C-terminal extremity or at their N-terminal extremity, so that they orient themselves in the same sense with regard to the corresponding metal ion **M**.

The first metal coordination moiety **21** can be identical or different from the second metal coordination moiety **31**. According to an embodiment, the first **21** and the second **31** metal coordination moieties are both independently selected from a natural amino-acid comprising at least one donating atom or atom group on its lateral chain. Such amino-acid can be selected among Cysteine (C), Methionine (M), Lysine (K), Arginine (R), Histidine (H), Tryptophane (W), Asparagine (N), Glutamine (Q), Serine (S), Threonine (T) and Tyrosine (Y). Preferably, one or both of the first **21** and the second **31** metal coordination moieties are independently selected from an amino-acid comprising at least one nitrogen atom on its lateral chain. Such amino-acids are thus preferably selected among Lysine (K), Arginine (R), Histidine (H), Tryptophane (W), Asparagine (N) and Glutamine (Q). In a most preferred embodiment, at least one of the first **21** and the second **31** metal coordination moiety comprises one or more heterocycle such as an imidazole, an indole or a related cyclic substituent. At least one of the first **21** and the second **31** metal coordination moiety can thus be selected among Histidine and Tryptophane.

In a preferred embodiment, both first **21** and second **31** metal coordination moieties denote a Histidine, wherein the imidazole ring coordinates with the metal ion **M**. Preferably, said metal ion **M** denotes a Cu (I) or a Cu (II) ion, most preferably a Cu (II) ion. Preferably, the Histidine is linked to the N-terminal extremity of the first peptide **20** to form the first metal coordination moiety **21** and a second Histidine is linked to the C-terminal extremity of the second peptide **30** to form the second metal coordination moiety **31**.

One or both of the first **21** and second **31** metal coordination moieties can be further modified. For example, free amine may be protonated so as to provide an ammonium group, free carboxylic acid groups may be acetylated or protected by a known protecting group, or transformed into an amide group. Other usual chemical transformations such as reductions, oxidations, protection and deprotection sequences can be performed on the metal coordination moieties and/or on the other amino-acids of the peptides.

It is understood that a given metal coordination moiety can be monodentate, bidentate or even tridentate, depending on the number of donating atoms it comprises. Although both the first and second metal coordination moieties can have the same degree of coordination, meaning that they are both monodentate or bidentate for example, this does not exclude that they have a different degree of coordination. For example, the first metal coordination moiety **21** can be monodentate while the second metal coordination moiety **31** is bidentate, or vice-versa, the first metal coordination moiety **21** can be bidentate while the second metal coordination moiety **31** is monodentate.

The coordination sphere of the metal ion **M** may comprise one or several other coordinated molecules such as water molecules, methanol molecules, ethanol molecules or any other solvent molecules or polar molecules present in the media. It can in addition comprise a reactive compound such as a reducing agent.

Both the first **20** and second **30** peptides comprise amino acids which remain neutral **23**, **33** under the pH conditions of the self-assembly **1**. In particular, the pH of the aqueous media wherein the self-assembly **1** is made is comprised between around 5.5 and around 8.5, preferably comprised between 6 and 8. The self-assembly **1** is preferably considered under neutral pH conditions, namely at pH comprised between 6.5 and 7.5. Neutral amino-acid **23**, **33** denote the amino-acids wherein the lateral chain remains uncharged under such neutral conditions. They are selected among the list of Glycine (G), Alanine (A), Isoleucine (I), Valine (V), Leucine (L), Phenylalanine (F), Proline (P), Methionine (M), Serine (S), Threonine (T), Tyrosine (Y), Cysteine (C), Glutamine (G), Asparagine (N) and Tryptophane (W). Other non-natural neutral amino-acids may be considered. Natural amino-acids comprised in the peptides can in addition be modified by one or several chemical reaction, provided that they remain neutral.

Both the first **20** and second **30** peptides comprise amino acids which are charged **22**, **32** under the pH conditions of the self-assembly **1**. The charged amino-acids **22**, **32** denotes those for which the lateral chain are either positively charged or negatively charged under the pH conditions. A negatively charged amino-acid can be selected among glutamic acid (E) and aspartic acid (D). A positively charged amino-acids can be selected among Lysine (K), Histidine (H) and Arginine (R). Other non-natural charged amino-acids may be considered. Natural amino-acids comprised in the peptides can in addition be modified by one or several chemical reactions so that they become charged or they remain charged amino-acids.

A given peptide, such as the first peptide **20** or the second peptide **30** or both preferably comprises 1, 2, 3 or four charged amino-acids **22**, **32**, being separated by a sequence of 2 to 18 neutral amino-acids **23**, **33**, or a sequence of 2 to 10 neutral amino-acids **23**, **33** or a sequence of 3, 4 or 5 neutral amino-acids **23**, **33**.

In a given self-assembly **1**, the first peptide **20** comprises at least one first charged amino-acid **22**, preferably 2 charged amino-acids **22**, and the remaining amino-acids denotes the first neutral amino-acids **23**. The first charged amino-acids **22** can be independently from each other positively charged or negatively charged. In a preferred embodiment all the first charged amino-acid **22** are either positively charged or negatively charged. Preferably, the first charged amino-acids **22** are positioned at both extremities of the first peptide **20**, or close to both extremities of the first peptide **20**, meaning at second or third position from each extremities, depending on the number of first amino-acids comprised in the first peptide **20**. In said given self-assembly **1**, the second peptide **30** comprises at least one second charged amino-acid **32**, preferably 2 charged amino-acids **32**, and the remaining amino-acids denote the second neutral amino-acids **33**. The second charged amino-acids **32** can be independently from each other positively charged or negatively charged. In a preferred embodiment all the second charged amino-acid **32** are either positively charged or negatively charged. Preferably, the second charged amino-acids **32** are positioned at both extremities of the second peptide **30**, or close to both extremities of the second peptide **30**, meaning at second or third position from each extremities, depending on the number of second amino-acids comprised in the second peptide **20**.

According to an embodiment, in a self-assembly **1** according to the present invention, the first **20** and second **30** peptides are arranged parallel to each other so as to form microfibrils. Where the C-terminal extremity of the first peptide **20** and the N-terminal extremity of the second peptide 30, or where the N-terminal extremity of the first peptide **20** and the C-terminal extremity of the second peptide **30** are provided with a metal coordination moiety, the first **20** and second **30** peptides are oriented in an antisense and antiparallel way. Most preferably, the first charged amino-acids **22** of the first peptide **20** are facing the second charged amino-acids **32** of the second peptide **30**, once the first **20** and the second **30** peptides are self-arranged parallel or antiparallel to each other, wherein the first charge amino-acids **22** and the corresponding second charged amino-acid **32** to which it facing have opposite charges, so that both first and second peptides remain attracted to one another by electrostatic interactions. Although the oppositely charged amino-acids **22**, **32** trigger the dimer formation of the self-assembly **1** and stabilises it, this does not excludes the presence of additional interactions such as hydrophobic interaction between neutral amino-acids, or π-π interactions.

According to one embodiment, the number of first neutral amino-acids **23** separating two first charged amino-acid **22** is identical to the number of second neutral amino-acids **33** separating two second charged amino-acids **32** so that all the first charged amino-acid **23** of the first peptide **20** faces all the second charged amino-acids **32** of the second peptide **32**. It is here understood that the position of the first charged amino-acid **22** in the first peptide **20** corresponds to position of the second charged amino-acid **32** in the second peptide once the first and the second peptides are bonded to the metal ion **M**.

It is noted that oppositely charged amino-acids **22**, **33**, arranged at the N and C terminals of the peptides improve the formation and the stability of the self-assembly **1**. Additional interactions such hydrophobic interactions between neutral amino-acids also improve the stability of the self-assembly **1.** Hydrogen bonds can occur between two peptide of a self-assembly **1** or between peptides of different self-assemblies, participating to supramolecular arrangements such as β-sheets.

Furthermore, the hydrophobic segment of the AAFF supports aggregation by hydrophobic and π-π interactions, which are commonly found in amyloid structures.

According to an example of the present invention, the first peptide **20** has the sequence :
**E1**: h-HKFFAAK-NH₂ wherein h-H denotes the histidine forming the first metal coordination moiety, K denotes Lysine as the first charged amino-acid, F denotes phenylalanine, A denotes alanine, wherein Lysine is positively charged;
the second peptide **30** has the sequence :
   **E2** : Ac-EFFAAEH-NH₂ wherein E denotes glutamic acid as a second charged amino-acid, F denotes phenyl alanine, A denotes Alanine and H denotes the Histidine forming the second metal coordination moiety, wherein the glutamic acid is negatively charged; and
   the metal ion **M** denotes Cu (II) ion.

It is understood that other amino-acid sequences can be envisaged provided that they correspond to the above described characteristics.

According to another embodiment shown in figure 2, the first **21** and second **31** metal coordination moieties are provided at positions different from the C-terminal or N-terminal extremities of the first **20** and/or second **30** peptides. The metal coordination moieties can for example correspond to a modified amino-acid of the first **20** and second **30** peptides, comprising at least one donating atom as above mentioned, adapted to bond to a metal ion **M**. In this particular case, the metal ion **M** is placed between the first and the second peptides in the self-assembly **1**. The self-assembly **1** can then spontaneously take different tridimentional structures, such as α-helix instead of β-sheets. It is understood that both first **20** and second **30** peptides still comprise charges amino-acids **22**, **32** facing each other and having opposite charged, which allow to maintain the self-assembly **1**. According to another arrangement, one of the metal coordination moiety can be at a one of the C-terminal and N-terminal extremities of one of the first **20** and second **30** peptide, and the other metal coordination moiety can be at a more central portion of the other peptide. The charged and neutral amino-acids can keep the same relative arrangement as above-described. In particular, the oppositely charged amino-acids **22**, **32** trigger the dimer formation of the self-assembly **1** and stabilises it.

According to another arrangement, the first **21** and second **31** metal coordination moieties are provided at positions different from the C-terminal or N-terminal extremities of only one of the first **20** and second **30** peptides. The charged and neutral amino-acids can keep the same relative arrangement as above-described. They are in particular stabilised through the above mentioned interactions. All the features above-described and applicable to such configurations are thus also part of these specific arrangements.

Although two peptides have been here described, the present self-assembly **1** can comprise more than two peptides, such as three or four peptides bonded to a metal ion **M**. In this particular case, all the metal coordination moieties can be selected as monodentate ligands.

The first **20** and second **30** peptides of a self-assembly **1** can interact with the first **20'** and second **30'** peptides of another self-assembly **1'** in the media to form a supramolecular structure. In addition to the oppositely charged amino-acids **22**, **32**, which trigger the dimer formation of the self-assembly **1**, interactions such as hydrogen bonds, hydrophobic and π-π interactions, between the self-assemblies **1** and **1'**, organise and stabilise a supramolecular arrangement. d through the formation of among the peptides scaffold. Furthermore, the hydrophobic segments, residues **23** and **33** supported self-assembly by hydrophobic and π-π interactions.

According to a preferred embodiment, the process of the present description relates the synthesis of a product of formula **P2** or a product of formula **P2'** or a mixture thereof:

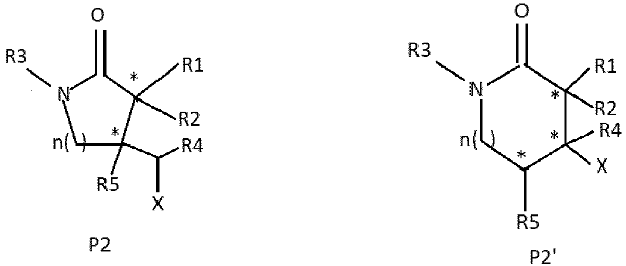

Starting from a product of formula **P1**,

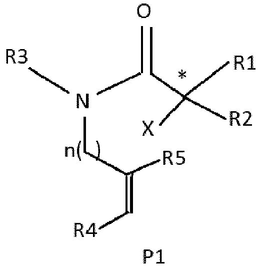

Wherein R₁, R₂, R₃, R₄, R₅, X and the star (*) are as above defined, and wherein n is an integer selected from 0, 1, 2, 3, 4 or 5.

The process involves or is operated through an Atom Transfer Radical Cyclisation under the conditions above-described.

In particular, it is performed in presence of a self-assembly **1** as above described.. More particularly, the self-assembly **1** above-described comprises a first peptide **20** having the sequence **E1**, a second peptide **30** having the sequence **E2** and Cu(II) ion as a metal ion **M**. More particularly, the metal ion is bounded to only one of the first **20** and the second **30** peptides, preferably at a central portion thereof, so that the self-assembly **1** takes an alpha helix arrangement.

Preferably, in the compounds **P2**, **P2'** and **P1**, R₁ and R₂ are independently selected from an hydrogen atom, a methyl group, or an halogen atom such as chlorine.

Preferably, in the compounds of formulae **P2**, **P2'** and **P1**, X denotes one of chlorine or bromine.

Preferably, in the compounds of formulae **P2**, **P2'** and **P1**, R₃ denotes a saturated or unsaturated alkyl group having from 1 to 4 carbon atoms, optionally substituted by one phenyl group, and/or optionally comprising one double bond. Preferably, R₃ is selected from a methyl group or a benzyl group.

Preferably, in the compounds of formulae **P2**, **P2'** and **P1**, R₄ and R₅ are independently from each other selected from Hydrogen, methyl, phenyl.

Preferably, in the compounds of formulae **P2**, **P2'** and **P1**, n denotes one of 0, 1 or 2.

The product of formula **P2** may be one of the followings:

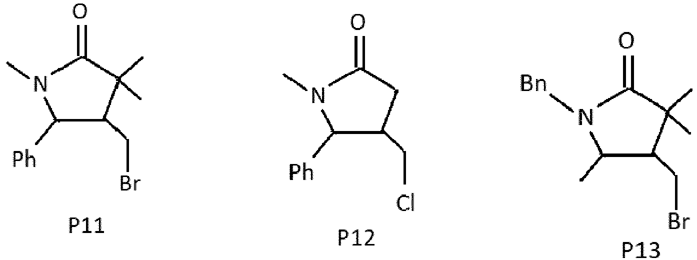

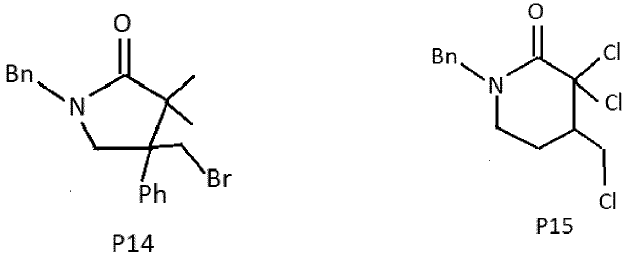

Depending on the substituents and the relative amount of the starting compounds, self-assembly and reducing agent, the ratio between **P2** / **P2'** is more than 50 / 50, preferably more than 70 /30, or more than 80 / 20, even more than 90 /10. The product **P2** may be obtained in more 98% or round 100%.

Depending on the value of n, the product **P2** designates a β-lactam, a γ-lactam, a ε-lactams or larger lactams analogs.

The reaction can be performed as a batch reaction. Alternatively, the reducing agent can be added to the reaction at a determined flow rate, either as a pure product or under solution. Flow rate of around 10 to 100 equivalents per hour can be used, such as around 20 to 50 equivalents per hour or around 30 equivalents per hour. The term "equivalent" denotes the molar ratio with regard to the metal ion amount.

Product **P2** is obtained with an enantiomeric excess of more than 10%, preferably more than 20%, more preferably more than 50%. The Product **P2** is obtained with 100% chemoselectivty. The chemo selectivity is largely improved when the reducing agent is added to the reaction at a controlled flow rate.

### Experimental part

### Chemicals:

Substrate stock solution 0.25 M in MetOH.
Peptide stock solution 10mM in buffer e.g. Tris pH 6.7.
CuSO₄*5H₂O : Stock 4mM in buffer.
Na Ascorbate stock solution freshly prepared in buffer: Stock 0.809 mg/100µl (10eq/100µl).

### Catalyst preparation:

In 1.5 mL vial equipped with a magnetic stirring bar add 50 µl of peptide 20 having the sequence E1 (0.5 µmol) and 100 µl CuSO₄*5H₂O (0.4 µmol). Stir the solution for 5 minutes and then add 50 ul peptide 30 having the sequence E2 (0.5 µmol). Stir at 37°C until fiber formation.

### Reaction set up:

To the 200 µl of catalyst fibers, add 10 µl of NaAsc (stock 0.809 mg/100 µl (10eq/100 µl), 1eq), stir for 10 minutes and then add 40 µl of substrate stock solution. Load the same Na Ascorbate solution in 5 ml syringe (10eq/100µl), and add the reducing agent with a speed of 100 µl/h (10eq/h) up to 100eq. The reaction does not requires any degassing step.

### Catalyst fiber lyophilization:

In 2.5 ml vial equipped with a magnetic stirring bar, add 500 um of peptide 20, having the sequence E1 (5 µmol) and 1000 ul CuSO₄*5H₂O (4 µmol). Stir the solution for 5 minutes and the add 500 ul peptide 30, having the sequence E2 (5 µmol). Stir at 37°C until fiber formation. In a new 1.5 ml vial Eppendorf tube, aliquote 200 µl of the suspension and lyophilize the peptide catalyst under vacuum. The solid fibers can be stored at room temperature or 4°C for long term storage and resuspended in 200 µl buffer to run catalysis.

### Example 1: Synthesis of 1-benzyl-4-(bromomethyl)-3,3-dimethylpyrrolidin-2-one C2

12,5 µmol of N-allyl-N-benzyl-2-bromo-2-methylpropanamide **C1** are mixed with 0,5 µmol of the catalyst based on the sequences **E1** and **E2** and Cu(II) ion in water, in presence of Tris buffer at pH = 6,7. The reaction is performed at 40°C during one hour. The catalyst is not degassed, meaning that it is an aerobic catalysis. The reaction provides the desired cyclic product **C2** and the corresponding product resulting from the reductive cyclisation **C3**.

C2: ¹H NMR (400MHz, Chloroform-d): *δ* 7.30-7.20 (m, 3H), 7.17-7.10 (m, 2H), 4.50-4.28 (m, 2H), 3.42 (dd, *J*=10.1, 4.8 Hz, 1H), 3.31 (dd, *J*=10.1, 7,6 Hz, 1H), 3.18 (dd, *J*=10.8, 10.1 Hz, 1Hz), 2.84 (dd, J=10.1, 8.6 Hz, 1H), 2.37 (dddd, J=10.8, 8.6, 7.6, 4.8 Hz, 1H), 1.19 (s, 3H), 0.94 (S, 3H).

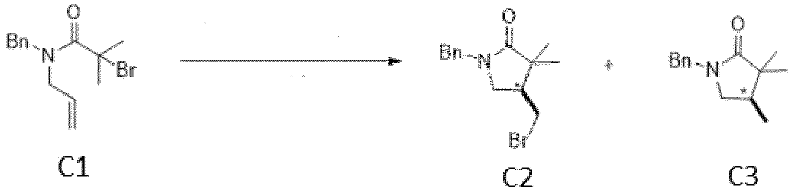

**Table 1:**

| **Entry** | **Catalyst** | **NaAsc (eq)** | **C2 (%)** | **C3 (%)** | **Conv. (%)** |
|---|---|---|---|---|---|
| **1** | CuSO₄ | 400 | 0 | 0 | 0 |
| **2** | Cu(TPMA) | 200 | 10 | 90 | 100 |
| **3** | Cu-E1/E2 | 200 | 56 | 44 | 87 |
| **4** | Cu-E1/E2 | 30 | 78,5 | 22,5 | 70 |
| **5** | Cu-E1/E2 | 5 | 100 | 0 | 4 |
| **6** | Cu-E1/E2 | 1 | 100 | 0 | 2 |
| **7** | Peptide E2 | 200 | 0 | 100 | 73 |
| **8** | Peptide E1 | 200 | 0 | 0 | 0 |

Table 1 above shows comparative data wherein Cu-E1/E2 denotes the catalyst above-described, NaAsc denotes sodium ascorbic acid as a reducing agent. Table 1 shows that **C2** is not obtained when using a traditional Cupper catalyst (entry 1, 2) or one of the peptides alone having the sequence **E1**, **E2** (entries 7, 8). Entries 3-6 shows that **C2** is obtained in the presence of the self-assembly 1 here described (enantiomeric excess 14%).

### Example 2: synthesis of 1-benzyl-4-(chloromethyl)-3,3-dichloropyrrolidin-2-one C2'

10 µmol of N-allyl-N-benzyl-2,2,2-trichloro-ethanamide **C1'** are mixed with 0.5 µmol of the catalyst based on the sequences **E1** and **E2** and Cu(II) ion in water, in the presence of Tris buffer at pH = 6,7. The reaction is performed at 40°C during 20 hours. The catalyst is not degassed, meaning that it is an aerobic catalysis. Ascorbic acid is used as a reducing gent according to a batch approach. The reaction provides the desired cyclic product **C2'** and the corresponding product resulting from the reductive cyclisation **C3'** as shown in table 2 below.

C2': ¹H NMR (400MHz, Chloroform-d): *δ* 7.45-7.27 (m, 3H), 7.28-7.21 (m, 5H), 4.63 (d, J=14.6 Hz, 1H), 4.45 (d, *J*=14.6 Hz, 1H), 4.02-3.90 (m, 1H), 3.73-3.58 (m, 1H), 3.53-3.39 (m, 1H), 3.14-3.02 (m, 2H).

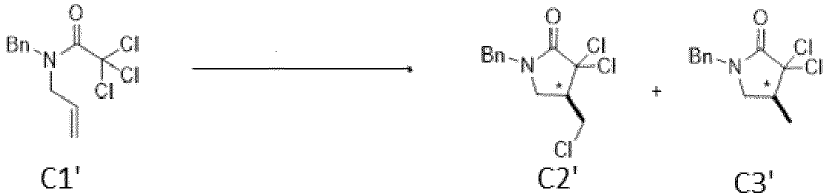

**Table 2:**

| **Entry** | **Catalyst** | **NaAsc (eq)** | **C2' (%)** | **C3' (%)** | **Conv. (%)** |
|---|---|---|---|---|---|
| **1** | CuSO₄ | 400 | 0 | 0 | 0 |
| **2** | Cu-E1/E2 | 5 | 100 | 0 | 15 |
| **3** | Peptide E2 | 200 | 0 | 100 | 100 |

Table 2 above shows comparative data wherein Cu-E1/E2 denotes the catalyst above-described, NaAsc denotes sodium ascorbic acid as a reducing agent. Table 2 shows that **C2'** is not obtained when using a traditional Cupper catalyst (entry 1) or the peptides alone having the sequence **E2** (entry 3). Entry 2 shows that **C2'** is obtained in the presence of the self-assembly **1** here described.

## Claims

1. Process for producing one of the products of formulae P20 and P20' or a mixture thereof 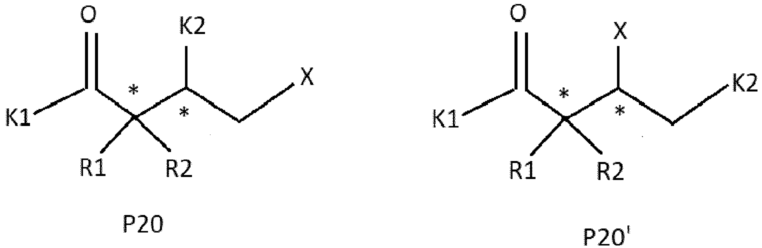 through an Atom Transfer Radical reaction selected from one of an Atom Transfer Radical Cyclisation ATRC, an Atom Transfer Radical Polymerisation ATRP and an Atom Transfer Radical Addition ATRA,
comprising the step of mixing a product of formula P10 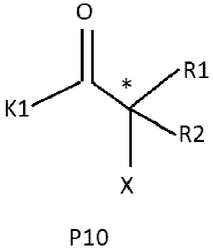 and a product of formula P10', 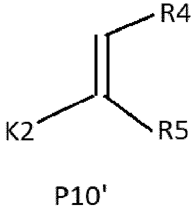 wherein :
• Wherein X denotes an halogen atom such as fluorine, bromine, chlorine or iodine, preferably fluorine, bromine or chlorine, most preferably, bromine or chlorine.
• R₁ and R₂ independently from each other denote hydrogen, an halogen atom selected from fluorine, chlorine, bromine, iodine, preferably, from chlorine or bromine, a linear or branched saturated or unsaturated alkyl group having from 1 to 10 carbon atoms, preferably from 1 to 5 carbon atoms, optionally substituted by 1 to 3 groups independently selected from OR₃, NR₃R_{3'}, SR₃, an aryl group, an heteroaryl group or a saturated or unsaturated cycloalkyl group having from 3 to 7 carbon atoms.
• R₃, R_{3'} independently from each other denote H, a linear or branched saturated or unsaturated alkyl group having from 1 to 10 carbon atoms, preferably from 1 to 5 carbon atoms, optionally substituted by 1 to 3 groups independently selected from an aryl group, an heteroaryl group or a saturated or unsaturated cycloalkyl group having from 3 to 7 carbon atoms.
• K1 and K2 independently from one another denote one of OR₃, NR₃R_{3'}, or SR₃, a linear or branched saturated or unsaturated alkyl group having from 1 to 10 carbon atoms, preferably from 1 to 5 carbon atoms, optionally substituted by 1 to 3 groups independently selected from OR₃, NR₃R_{3'}, and SR₃ and wherein one of the carbon atom of said linear or branched saturated or unsaturated alkyl group is optionally replaced by an oxygen or a sulphur atom or by a group NR₃
• The star (*) denotes a chiral centre where the corresponding substituents are all different.
In the presence of a reducing agent and a peptide based catalyst in a solvent **characterized in that**:
- said solvent is water,
- said peptide based catalyst comprises a self-assembly (1) of at least:
∘ a first peptide (20),
∘ a second peptide (30), and
∘ a metal ion (M),
and **in that** said Atom Transfer Radical reaction is performed at a temperature comprised between 25°C and 45°C.

2. Process according to claim 1, wherein said first peptide (20) comprises at least one first metal coordination moiety (21) and said second peptide (30) comprises at least one second metal coordination moiety (31), or wherein only one of said first peptide (20) and second peptide (30) comprises at least one metal coordination moiety, the metal ion (M) being coordinated to said metal coordination moieties.

3. Process according to claims 1 or 2, wherein said metal ion (M) is bonded to one terminal portion or at a central portion of at least one of said first (20) and second (30) peptides.

4. Process according to one of claims 1 to 3, wherein said first peptide (20) comprises at least one first charged amino-acid (22) and said second peptide (30) comprises at least one second charged amino-acid (32) arranged so that said first (20) and second (30) peptides interact to each other to form and maintain said self-assembly (1) at a pH comprised between about 6 and about 8 in an aqueous media.

5. Process according to one of claims 1 to 4, wherein both the first (20) and second (30) peptides comprise amino acids which remain neutral (23, 33) under the pH conditions of the process.

6. Process according to one of claims 4 or 5, wherein at least some of the first charged amino-acids (22) of the first peptide (20) are facing the second charged amino-acids (32) of the second peptide (30), wherein the first (20) and the second (30) peptides are self-arranged parallel or antiparallel to each other, wherein the first charge amino-acids (22) and the corresponding second charged amino-acid (32) to which it is facing have opposite charges, so that both first and second peptides remain attracted to one another by electrostatic interactions.

7. Process according to one of claims 5 or 6, wherein said neutral amino-acids (23, 33) are arranged between the corresponding charged amino-acids (22, 32), the number of first neutral amino-acids (23) separating two first charged amino-acid (22) being identical to the number of second neutral amino-acids (33) separating two second charged amino-acids (32) so that all the first charged amino-acid (23) of the first peptide (20) face all the second charged amino-acids (32) of the second peptide (32).

8. Process according to one of claims 1 to 7, wherein said reducing agent is sodium ascorbate, and is added in the reaction in excess with regards to the metal ion (M).

9. Process according to one of claims 1 to 8, wherein said metal ion is a cupper ion selected from Cu(I) and Cu(II).

10. Process according to one of claims 1 to 9, wherein the first peptide (20) has the sequence :
**E1**: h-HKFFAAK-NH₂ wherein h-H denotes the histidine forming the first metal coordination moiety, K denotes Lysine as the first charged amino-acid, F denotes phenylalanine, A denotes alanine, wherein Lysine is positively charged;
the second peptide (30) has the sequence :
**E2** : Ac-EFFAAEH-NH₂ wherein E denotes glutamic acid as a second charged amino-acid, F denotes phenyl alanine, A denotes Alanine and H denotes the Histidine forming the second metal coordination moiety, wherein the glutamic acid is negatively charged.

11. Process according to one of claims 1 to 10, wherein K1 and K2 form together a linker so that the compounds of formulae P10 and P10' form one compound of formula P10", 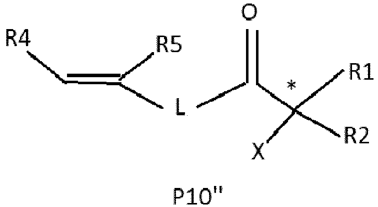 Wherein
• L denotes a linear or branched saturated or unsaturated alkyl group having from 1 to 10 carbon atoms, preferably from 1 to 5 carbon atoms, optionally substituted by 1 to 3 groups independently selected from OR₃, NR₃R_{3'}, and SR₃, and/or wherein one of the carbon atom of said linear or branched saturated or unsaturated alkyl group is optionally replaced by an oxygen or a sulphur atom or by a group NR₃,
• wherein X, R₁, R₂, R₃, R_{3'}, R₄, R₅ and the star (*) are has above defined.
Wherein said Atom Transfer Radical reaction is an Atom Transfer Radical Cyclisation leading to a resulting product which is a compound of formula P21. 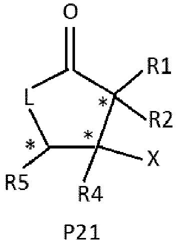 Wherein L, X, R₁, R₂, R₃, R₄, R₅ and the stars (*) are has above defined.

12. Process according to claim 11, wherein the compound of formula P10" is a compound of formula P1 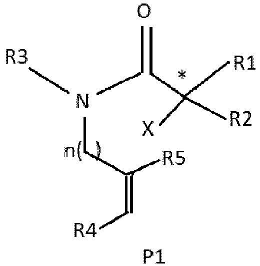 and wherein the resulting product is a compound of formula P 2 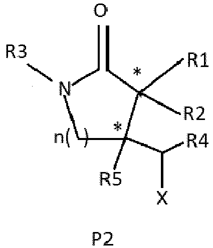 Wherein R₁, R₂, R₃, R₄, R₅, X and the star (*) are as above defined, and wherein n is an integer selected from 0, 1, 2, 3, 4 or 5, preferably from 0, 1, 2 or 3.

13. Process according to one of claims 1 to 12, wherein the final product is one of the following

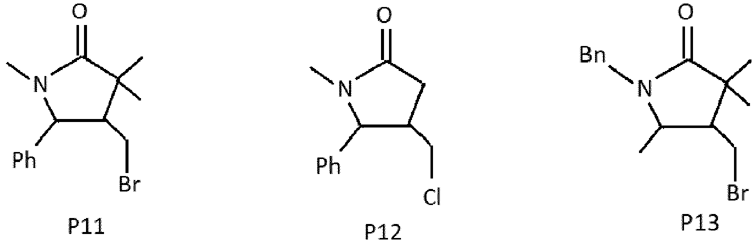

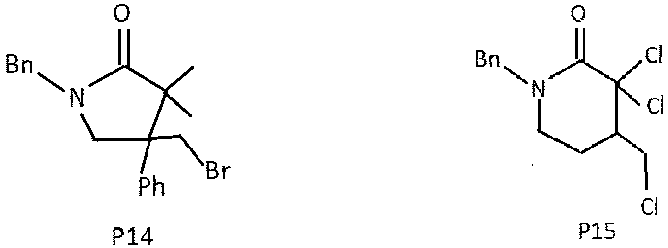

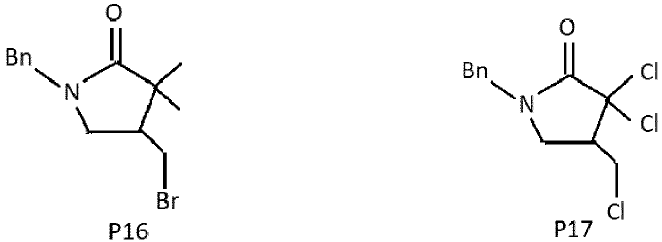

14. A self-assembly (1) of at least two peptides and a metal ion (M) comprising:
- a first peptide (20),
- a second peptide (30), and
- a metal ion (M),
**characterized in that** the first peptide (20) comprises one or more first metal coordination moiety (21), **in that** said second peptide (30) comprises one or more second metal coordination moiety (31), wherein the metal ion (M) is coordinated to at least one or to only one of said first coordination moiety (21) and the second coordination moiety (31), wherein said first peptide (20) comprises at least one first charged amino-acid (22) and said second peptide (30) comprises at least one second charged amino-acid (32) arranged so that said first (20) and second (30) peptides interact to each other to form and maintain said self-assembly (1) at a pH comprised between about 6 and about 8 in an aqueous media.

15. Self-assembly according to claim 14 wherein said one or more first metal coordination moiety (21) is arranged at a central portion of said first peptide (20) and/or in that said second metal coordination moiety (31) is arranged at one of the C-terminal and N-terminal extremities of the second peptide (30) or at a central portion of said second peptide (30), wherein said at least one first charged amino-acid (22) faces said at least one second charged amino-acid (32), wherein the at least one first (22) and the at least one second (32) charged amino-acids facing each other have opposite charges.
